# EUROPEAN PATENT APPLICATION

(11) **EP 4 169 889 A1**
(43) Date of publication of application: **26.04.2023**
(21) Application number: 21204127.1
(22) Date of filing: 22.10.2021
(51) Int. Cl.: C05C 9/02, B01J 2/16

(54) **CONTINUOUS METHODS FOR FORMING METHYLENE UREA-ISOBUTYLENE DIUREA GRANULES**

(71) Applicant: SABIC Global Technologies, B.V., 4612 PX Bergen op Zoom (NL)
(72) Inventor: ACHANATH, Radha, Bangalore (IN); KUMAR, Arvind, Bangalore (IN); AT, Kavya, Bangalore (IN)
(74) Representative: Dehns

(57) **Abstract**

A continuous method for making granules of methylene urea-isobutylene diurea (MU-IBDU) includes: injecting an MU-IBDU slurry including water and MU-IBDU particles into a fluid bed granulator, wherein the fluid bed granulator includes seed particles; injecting air into the fluid bed granulator to fluidize the seed particles in an air stream; spraying the MU-IBDU slurry over the fluidized seed particles to form granules of MU-IBDU; and removing the granules of MU-IBDU from the fluid bed granulator. Fertilizer compositions including the MU-IBDU granules are also described.

## Description

### FIELD OF THE DISCLOSURE

The present disclosure relates to continuous methods for forming granules including methylene urea-isobutylene diurea (MU-IBDU).

### BACKGROUND OF THE DISCLOSURE

Conventional processes for granulation of urea condensate products typically utilize a powder/slurry route including a pan/disc and drum as granulation equipment. These processes have various shortcomings. They are laborious, requiring drying of the synthesized product to obtain a powder which is then re-moisturized for the pan granulator. Also a high concentration of synthesized product is required for it to be suitable for the drum granulator. Such processes are also human labor intensive; shifting of the dried powder required more human intervention and additional solid handling equipment. Further, pan granulation can only be accomplished in a batch process due to the complexity of re-moisturization of the dried powder. Finally, conventional processes have quality problems. The bulk density/ crushing strength of the granular product is low when a pan granulator is used. Drum granulation processes offer slightly improved quality, but still not as high as desired.

U.S. Patent Application Publication No. 2009/0165515 describes a granular slow-release nitrogenous fertilizer which is less in the release velocity of a nitrogen component than a conventional granular slow-release nitrogenous fertilizer and which is excellent in long-term persistence and the sustained releasability of the nitrogen component. The granular slow-release nitrogenous fertilizer is prepared by granulating a mixture of a urea-aldehyde condensate and an oxidized wax. The content of the oxidized wax is preferably three to 25 weight percent and more preferably five to 15 weight percent. The oxidized wax contains polar groups and therefore has extremely excellent compatibility with the urea-aldehyde condensate. Therefore, the urea-aldehyde condensate, which is sparingly soluble, is extremely uniformly dispersed in the granular slow-release nitrogenous fertilizer according to the present invention; hence, the release velocity of the urea-aldehyde condensate into soil is low.

U.S. Patent No. 5,039,328 describes a process for producing a granular slow-acting nitrogenous fertilizer, which comprises blending an aqueous methylol urea solution to a urea-isobutyraldehyde condensate powder or a mixture thereof with a urea-formaldehyde condensate powder, and granulating the resulting blend while subjecting the methylol urea to dehydration condensation under an acidic condition to form a urea-formaldehyde condensate.

U.S. Patent Application Publication No. 2003/0154754 describes a method for making nitrogenous and complex fertilizers, in granule form, suitable for slowly releasing nitrogen, comprising the steps of preparing an aqueous urea/formaldehyde dispersion with a molar ratio from 0.8:1 to 2:1, adding an aqueous catalyzer solution in a weight ratio from 0.1 to 4%, controlling the pH of the aqueous dispersion to hold it within a range from 4.0 to 7.0, and supplying the aqueous dispersion to a recirculating granulating device, as a growth seed.

All of these conventional methods for making fertilizer compositions suffer from various shortcomings, however.

These and other shortcomings are addressed by aspects of the present disclosure.

### SUMMARY

Aspects of the disclosure relate to a continuous method for making granules of methylene urea-isobutylene diurea (MU-IBDU) including: injecting an MU-IBDU slurry including water and MU-IBDU particles into a fluid bed granulator, wherein the fluid bed granulator includes seed particles; injecting air into the fluid bed granulator to fluidize the seed particles in an air stream; spraying the MU-IBDU slurry over the fluidized seed particles to form granules of MU-IBDU; and removing the granules of MU-IBDU from the fluid bed granulator. Fertilizer compositions including the MU-IBDU granules are also described.

### BRIEF DESCRIPTION OF THE FIGURES

In the drawings, which are not necessarily drawn to scale, like numerals may describe similar components in different views. Like numerals having different letter suffixes may represent different instances of similar components. The drawings illustrate generally, by way of example, but not by way of limitation, various aspects discussed in the present document.
FIG. 1 is a schematic of a fluidization bed apparatus for a continuous method for making granules of methylene urea-isobutylene diurea (MU-IBDU) according to aspects of the disclosure.
FIGS. 2A and 2B are optical microscopy images of MU-IBDU granules formed according to aspects of the disclosure.

### DETAILED DESCRIPTION

The present disclosure can be understood more readily by reference to the following detailed description of the disclosure and the Examples included therein. In various aspects, the present disclosure pertains to a continuous method for making granules of methylene urea-isobutylene diurea (MU-IBDU) including: injecting an MU-IBDU slurry including water and MU-IBDU particles into a fluid bed granulator, wherein the fluid bed granulator includes seed particles; injecting air into the fluid bed granulator to fluidize the seed particles in an air stream; spraying the MU-IBDU slurry over the fluidized seed particles to form granules of MU-IBDU; and removing the granules of MU-IBDU from the fluid bed granulator. Fertilizer compositions including the MU-IBDU granules are also described.

Before the present compounds, compositions, articles, systems, devices, and/or methods are disclosed and described, it is to be understood that they are not limited to specific synthetic methods unless otherwise specified, or to particular reagents unless otherwise specified, as such can, of course, vary. It is also to be understood that the terminology used herein is for the purpose of describing particular aspects only and is not intended to be limiting.

Various combinations of elements of this disclosure are encompassed by this disclosure, e.g., combinations of elements from dependent claims that depend upon the same independent claim.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; and the number or type of aspects described in the specification.

All publications mentioned herein are incorporated herein by reference to disclose and describe the methods and/or materials in connection with which the publications are cited.

### Definitions

It is also to be understood that the terminology used herein is for the purpose of describing particular aspects only and is not intended to be limiting. As used in the specification and in the claims, the term "comprising" can include the aspects "consisting of' and "consisting essentially of." Unless defined otherwise, all technical and scientific terms used herein have the same meaning as commonly understood by one of ordinary skill in the art to which this disclosure belongs. In this specification and in the claims which follow, reference will be made to a number of terms which shall be defined herein.

As used in the specification and the appended claims, the singular forms "a," "an" and "the" include plural referents unless the context clearly dictates otherwise. Thus, for example, reference to "a seed particle" includes mixtures of two or more different types of seed particles.

As used herein, the term "combination" is inclusive of blends, mixtures, alloys, reaction products, and the like.

Ranges can be expressed herein as from one value (first value) to another value (second value). When such a range is expressed, the range includes in some aspects one or both of the first value and the second value. Similarly, when values are expressed as approximations, by use of the antecedent 'about,' it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "about" and "at or about" mean that the amount or value in question can be the designated value, approximately the designated value, or about the same as the designated value. It is generally understood, as used herein, that it is the nominal value indicated ±10% variation unless otherwise indicated or inferred. The term is intended to convey that similar values promote equivalent results or effects recited in the claims. That is, it is understood that amounts, sizes, formulations, parameters, and other quantities and characteristics are not and need not be exact, but can be approximate and/or larger or smaller, as desired, reflecting tolerances, conversion factors, rounding off, measurement error and the like, and other factors known to those of skill in the art. In general, an amount, size, formulation, parameter or other quantity or characteristic is "about" or "approximate" whether or not expressly stated to be such. It is understood that where "about" is used before a quantitative value, the parameter also includes the specific quantitative value itself, unless specifically stated otherwise.

Disclosed are the components to be used to prepare the compositions of the disclosure as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific aspect or combination of aspects of the methods of the disclosure.

References in the specification and concluding claims to parts by weight of a particular element or component in a composition or article, denotes the weight relationship between the element or component and any other elements or components in the composition or article for which a part by weight is expressed. Thus, in a compound including 2 parts by weight of component X and 5 parts by weight component Y, X and Y are present at a weight ratio of 2:5, and are present in such ratio regardless of whether additional components are included in the compound.

A weight percent of a component, unless specifically stated to the contrary, is based on the total weight of the formulation or composition in which the component is included.

As used herein the terms "weight percent," "wt%," and "wt. %," which can be used interchangeably, indicate the percent by weight of a given component based on the total weight of the composition, unless otherwise specified. That is, unless otherwise specified, all wt% values are based on the total weight of the composition. It should be understood that the sum of wt% values for all components in a disclosed composition or formulation are equal to 100.

Unless otherwise stated to the contrary herein, all test standards are the most recent standard in effect at the time of filing this application.

Each of the materials disclosed herein are either commercially available and/or the methods for the production thereof are known to those of skill in the art.

It is understood that the compositions disclosed herein have certain functions. Disclosed herein are certain structural requirements for performing the disclosed functions and it is understood that there are a variety of structures that can perform the same function that are related to the disclosed structures, and that these structures will typically achieve the same result.

### Continuous Method for Making Granules of MU-IBDU

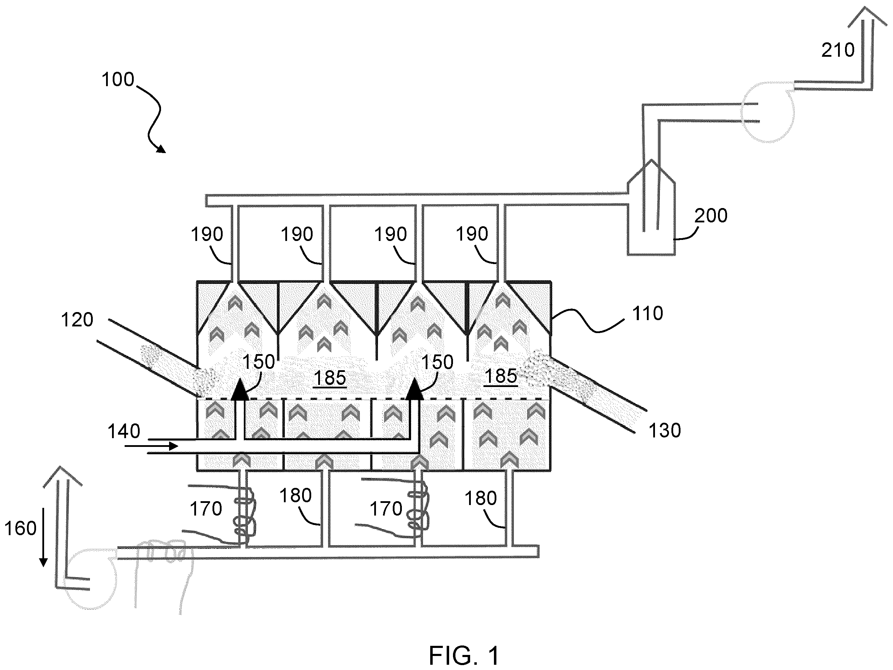

With reference to FIG. 1, aspects of the disclosure relate to a continuous method for making granules of methylene urea-isobutylene diurea (MU-IBDU). FIG. 1 illustrates an exemplary fluid bed granulator apparatus 100 which includes a fluidization bed 110, an inlet 120 for seed entry and an outlet 130 for removal of MU-IBDU granule product. An MU-IBDU slurry including water and MU-IBDU particles is injected into the fluidization bed 110 through a slurry inlet 140 and nozzles 150. The MU-IBDU slurry is maintained at, e.g., a temperature ranging from 60-110 degrees Celsius (°C) with efficient agitation to prevent gel formation. The heated MU-IBDU hot slurry is injected into the granulator at a steady flow rate to form a layer-by-layer coating over the seed loaded in the fluidization bed 110.

Fluidization air (e.g., atmospheric air) is injected into the fluidization bed 110 through air inlet 160. The fluidization air may be heated (at 170) if desired for particle size modification. In some aspects the fluidization air is both heated (170) and either cooled or maintained at atmospheric temperature (180) to control particle size distribution in individual chambers 185 of the fluidization bed 110. Fluidization air is removed from the fluidization bed 110 through air outlets 190 and filtered in a cyclone separator 200 to remove fertilizer dust before being vented back to the atmosphere 210. As shown in FIG. 1, the fluidization bed 110 includes four chambers 185, but it will be recognized that fewer or more chambers 185 could be included.

During operation of the apparatus 100 the MU-IBDU slurry is injected into the fluidization bed 110 where the seeds located therein are coated with the slurry. After a certain residence time the coated seeds move to the next chamber 185 where the fluidizing air is kept at lower temperature to allow the coated seeds/granule to harden and removed stickiness. The fluidization air entering at 170 and 180 helps to maintain the seed particles in a fluidized state, remove crystallization heat, remove moisture, and transport seeds from one chamber 185 to another. In addition, the fluidization air separates fine dust from the granules. Exemplary operating conditions in the apparatus 100 are listed below in Table 1:

**Table 1 - Operating Conditions of Fluid Bed Granulator Apparatus**

| **Description** | **Units** | **Range** | **Nominal value/range** |
|---|---|---|---|
| MU-IBDU slurry temperature (at 130) | °C | 60 to 110 | ∼70 |
| MU-IBDU slurry feed rate (at 130) | L/hr | >0 to 4 | ∼4 |
| Drying chamber fluidization air temperature (at 180) | °C | 35 to 90 | 80 to 90 |
| Feed Chamber Fluidization air temp (at 170) | °C | 70 to 170 | 120 to 150 |
| Atomization air (with MU-IBDU slurry, at 130) | °C | 70 to 140 | 90 to 110 |
| Atomization air pressure (with MU-IBDU slurry, at 130) | kg/cm²·g | < 1.0 | < 0.5 |
| Feed bed temperature (in first and third chambers) | °C | 80 to 140 | 100 to 110 |
| Drying bed temperature (in second and fourth chambers) | °C | 50 to 100 | 90-100 |
| Vacuum (at air outlets 190) | mbar | -5 to 50 | -5 |
| Air flow (at 160, for a 4-chamber bed including -20 kg of seed particles) | m³/hr | 300 to 1400 | 700 to 900 (∼200 per bed) |

| | | | |
|---|---|---|---|
| L/hr = liters per hour; kg/cm²·g = kilogram per square centimeter-gram; mbar = millibar; m³/hr = cubic meters per hour | | | |

Accordingly, aspects of the disclosure relate to a continuous method for making granules of methylene urea-isobutylene diurea (MU-IBDU), the continuous method including: injecting an MU-IBDU slurry including water and MU-IBDU particles into a fluid bed granulator, wherein the fluid bed granulator includes seed particles; injecting air into the fluid bed granulator to fluidize (i.e., suspend) the seed particles in an air stream; spraying the MU-IBDU slurry over the fluidized seed particles to form granules of MU-IBDU; and removing the granules of MU-IBDU from the fluid bed granulator.

In some aspects the granules of MU-IBDU have a particle size of from about 1.5 millimeter (mm) to about 5 mm. In further aspects the granules of MU-IBDU have a particle size of from about 2 mm to about 4 mm.

The granules of MU-IBDU may have improved crush strength as compared to MU-IBDU granules formed according to conventional methods. In certain aspects the granules of MU-IBDU have a crush strength of from about 1.5 to about 5 kg/granule, or from about 1.5 to about 3 kg/granule, or from about 1.5 to about 2.5 kg/granule, or at least about 1.5 kg/granule, or at least about 2.0 kg/granule.

The fluid bed granulator may include at least 2 chambers including a first chamber and a second chamber. Air may be injected into the first chamber at a first temperature of from about 70 °C to 170 °C, and into the second chamber at a second temperature of from about 35 °C to 90 °C. The second temperature may be lower than the first temperature in some aspects. The higher temperature in the first chamber helps to coat the seed particles with the MU-IBDU; the lower temperature in the second chamber helps to dry the MU-IBDU granules.

In certain aspects the MU-IBDU slurry is injected into the fluid bed granulator at a temperature of from about 30 °C to about 110 °C. In further aspects the MU-IBDU slurry is injected into the fluid bed granulator at a temperature of from about 60 °C to about 110, or at a temperature of from about 60 °C to 80 °C, or about 70 °C.

In further aspects the MU-IBDU slurry is mixed with atomizing air prior to or at the time of injecting the MU-IBDU slurry into the fluid bed granulator. The atomizing air has a temperature of from about 70 °C to 140 °C in some aspects, or a temperature of from about 90 °C to about 110 °C.

The granules of MU-IBDU have a ratio of MU to IBDU of from about 90:10 to about 25:75 in some aspects. For example, the granules of MU-IBDU may include 90 wt% MU and 10 wt% IBDU, or 85 wt% MU and 15 wt% IBDU, or 75 wt% MU and 25 wt% IBDU, or 50 wt% MU and 50 wt% IBDU, or 25 wt% MU and 75 wt% IBDU.

The seed particles include MU-IBDU in some aspects. In other aspects the seed particle includes another component other than MU-IBDU. If a seed particle other than MU-IBDU is used a portion of the initial product may need to be discarded, which helps to maintain the purity of the desired MU-IBDU granules.

In some aspects the step of removing the granules of MU-IBDU from the fluid bed granulator includes at least partially discharging the granules of MU-IBDU from the fluid bed granulator and passing the partially discharged granules of MU-IBDU through a screen to collect particles in a desired particle size range (e.g., 1.5-5.0 mm or 2-4 mm as described herein).

The method may further include returning undersized granules of MU-IBDU to the fluid bed granulator as MU-IBDU seed particles. Undersized granules may include, e.g., granules having a particle size less than 1.5 mm or less than 2.0 mm. In yet further aspects the method includes crushing oversized granules of MU-IBDU to form smaller sized granules and returning the smaller sized granules to the fluid bed granulator as MU-IBDU seed particles. Oversized granules may include, e.g., granules having a particle size of greater than 5.0 mm, greater than 4.5 mm, or greater than 4 mm.

Methods for forming MU-IBDU granules according to aspects of the disclosure allow the synthesized MU-IBDU slurry to be fed directly into the granulator; thereby avoiding separate evaporation and drying processes. Having achieved this advantage, solid handling steps are avoided, including the need for solid handling equipment and human labor. The methods further allow for layer-by-layer coating in a liquid state. The subsequently formed and dried granule is compact and has improved morphology and crush strength properties. Further, the method may be operated as a continuous process and can be easily commercialized.

### Fertilizer Compositions Including the MU-IBDU Granules

Some aspects of the disclosure relate to a fertilizer composition including the granules of MU-IBDU formed according to the methods described herein. The fertilizer composition may include other components in addition to the MU-IBDU granules, including but not limited to an additional fertilizer, a micronutrient, a secondary nutrient, a trace element, a plant protection agent, a filler, an organic additive, or a combination thereof.

Examples of additional fertilizers include, but are not limited to, nitrogen fertilizers, phosphate fertilizers, alkaline fertilizers, potassium and/or magnesium containing fertilizers, and/or manure, and/or secondary nutrients, and/or trace elements. Examples of nitrogen fertilizers include organic fertilizer containing nitrogen, such as crotonylidene diurea, oxamide, melamine, substituted triazones, ethylene diurea, triuret, and any mixtures of thereof. For example, the other fertilizers can additionally contain urea or nitrogen, potassium, phosphorus, and/or magnesium in the form of inorganic salts, or mixtures thereof. Easily soluble nitrogen components are, for example, ammonium nitrate, ammonium sulfate, or urea. Other salts that can be used are, for example, monoammonium phosphate, diammonium phosphate, potassium sulfate, potassium chloride, magnesium sulphate, calcium superphosphate, disodium hydrogen phosphate, ferric chloride, manganous chloride, calcium chloride, magnesium phosphate, ammonia, and potassium oxide. The other fertilizers can contain single-nutrient, multi -nutrient, and other possible fertilizer ingredients, for example, which contain nutrients such as nitrogen, potassium, or phosphorus, individually or in combination. In some instances, the nutrients are in the form of their salts. Examples of these are nitrogen and phosphorous fertilizers (NP), nitrogen and potassium fertilizers (NK), potassium and phosphorous fertilizers (PK), and nitrogen, phosphorus, and potassium fertilizers (NPK), lime nitrate of ammonium, ammonia sulfate, ammonia sulfa-nitrate, and urea. [0050] Examples of secondary nutrients include, but are not limited to, Ca, S, and Mg. Trace elements, for example selected from among Fe, Mn, Cu, Zn, Mo, B, or mixtures thereof, can also be present for example in the form of inorganic salts. The amounts of secondary nutrients or trace elements in the mixed fertilizer can, for example, be chosen in the range from 0.5 to 5 wt. %, based upon the total weight of the mixed fertilizer. [0051] Examples of plant protection agents include but are not limited to insecticides, fungicides, growth regulators, nitrification inhibitors, and any mixtures of them. Examples of fillers include but are not limited to clay, peat, etc.

Various combinations of elements of this disclosure are encompassed by this disclosure, e.g., combinations of elements from dependent claims that depend upon the same independent claim.

### Aspects of the Disclosure

In various aspects, the present disclosure pertains to and includes at least the following aspects.

Aspect 1. A continuous method for making granules of methylene urea-isobutylene diurea (MU-IBDU), the continuous method comprising:
injecting an MU-IBDU slurry comprising water and MU-IBDU particles into a fluid bed granulator, wherein the fluid bed granulator comprises seed particles;
injecting air into the fluid bed granulator to fluidize the seed particles in an air stream;
spraying the MU-IBDU slurry over the fluidized seed particles to form granules of MU-IBDU; and
removing the granules of MU-IBDU from the fluid bed granulator.

Aspect 2. The continuous method according to Aspect 1, wherein the granules of MU-IBDU have a particle size of from about 1.5 millimeter (mm) to about 5 mm.

Aspect 3. The continuous method according to Aspect 1 or 2, wherein the granules of MU-IBDU have a crush strength of at least 1.5 kilogram (kg) per granule as tested with a Chatillon 225 series digital force tester.

Aspect 4. The continuous method according to any of Aspects 1 to 3, wherein the fluid bed granulator comprises at least 2 chambers comprising a first chamber and a second chamber, and wherein the air is injected into the first chamber at a first temperature of from about 70 °C to 170 °C and the air is injected into the second chamber at a second temperature of from about 35 °C to 90 °C, wherein the second temperature is lower than the first temperature.

Aspect 5. The continuous method according to any of Aspects 1 to 4, wherein the MU-IBDU slurry is injected into the fluid bed granulator at a temperature of from about 30 °C to about 110 °C.

Aspect 6. The continuous method according to any of Aspects 1 to 5, further comprising mixing the MU-IBDU slurry with atomizing air prior to or at the time of injecting the MU-IBDU slurry into the fluid bed granulator.

Aspect 7. The continuous method according to Aspect 6, wherein the atomizing air has a temperature of from about 70 °C to 140 °C.

Aspect 8. The continuous method according to any of Aspects 1 to 7, wherein the granules of MU-IBDU have a ratio of MU to IBDU of from about 90:10 to about 25:75.

Aspect 9. The continuous method according to any of Aspects 1 to 8, wherein the seed particles comprise MU-IBDU.

Aspect 10. The continuous method according to any of Aspects 1 to 9, wherein the step of removing the granules of MU-IBDU from the fluid bed granulator comprises at least partially discharging the granules of MU-IBDU from the fluid bed granulator and passing the partially discharged granules of MU-IBDU through a screen to collect particles in a desired particle size range.

Aspect 11. The continuous method according to Aspect 10, further comprising returning undersized granules of MU-IBDU to the fluid bed granulator as MU-IBDU seed particles.

Aspect 12. The continuous method according to Aspect 10, further comprising crushing oversized granules of MU-IBDU to form smaller sized granules and returning the smaller sized granules to the fluid bed granulator as MU-IBDU seed particles.

Aspect 13. A fertilizer composition comprising the granules of MU-IBDU formed according to the method of any of Aspects 1 to 12.

Aspect The fertilizer composition according to Aspect 13, further comprising an additional fertilizer, a micronutrient, a secondary nutrient, an organic additive, or a combination thereof.

Aspect 15. The fertilizer composition according to Aspect 13 or 14, wherein the granules of MU-IBDU have a particle size of from about 1.5 mm to about 5 mm.

Aspect 16. The fertilizer composition according to any of Aspects 13 to 15, wherein the granules of MU-IBDU have a crush strength of at least 1.5 kg per granule as tested with a Chatillon 225 series digital force tester.

Aspect 17. The fertilizer composition according to any of Aspects 13 to 16, wherein the granules of MU-IBDU have a ratio of MU to IBDU of from about 90:10 to about 25:75.

### EXAMPLES

The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric. Unless indicated otherwise, percentages referring to composition are in terms of wt%.

There are numerous variations and combinations of reaction conditions, e.g., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

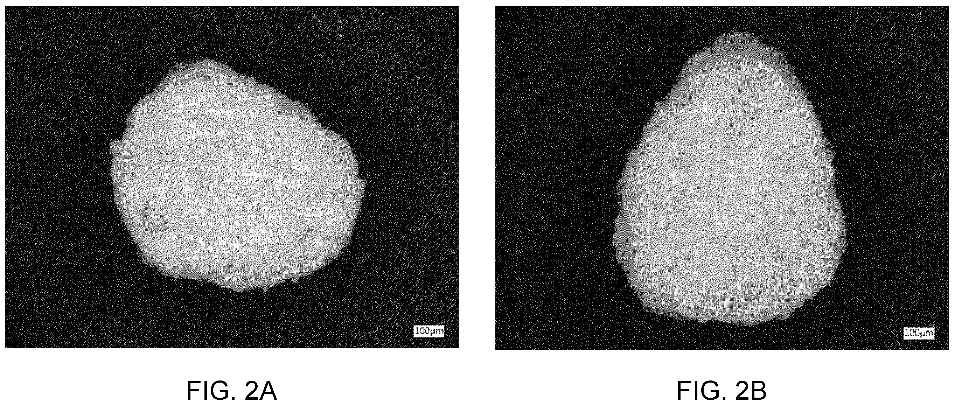

MU-IBDU granules were prepared according to methods described herein using the nominal values/ranges set forth in Table 1 above. Optical microscopy images of MU-IBDU exemplary granules are shown in FIGS. 2A and 2B. The granules have a relatively smooth surface as compared to MU-IBDU granules formed according to conventional methods. In addition, they have a compact morphology and do not have a hollow structure. Further, the MU-IBDU granules included about 75% methylene urea and about 25% isobutylene diurea, had a size of about 2-4 mm, a crush strength of 2.0 kilograms per granule and a total nitrogen content of about 38.4 wt%.

The above description is intended to be illustrative, and not restrictive. For example, the above-described examples (or one or more aspects thereof) may be used in combination with each other. Other aspects can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed aspect. Thus, the following claims are hereby incorporated into the Detailed Description as examples or aspects, with each claim standing on its own as a separate aspect, and it is contemplated that such aspects can be combined with each other in various combinations or permutations. The scope of the disclosure should be determined with reference to the appended claims, along with the full scope of equivalents to which such claims are entitled.

## Claims

1. A continuous method for making granules of methylene urea-isobutylene diurea (MU-IBDU), the continuous method comprising:
injecting an MU-IBDU slurry comprising water and MU-IBDU particles into a fluid bed granulator, wherein the fluid bed granulator comprises seed particles;
injecting air into the fluid bed granulator to fluidize the seed particles in an air stream;
spraying the MU-IBDU slurry over the fluidized seed particles to form granules of MU-IBDU; and
removing the granules of MU-IBDU from the fluid bed granulator.

2. The continuous method according to claim 1, wherein the granules of MU-IBDU have a particle size of from about 1.5 millimeter (mm) to about 5 mm.

3. The continuous method according to claim 1 or 2, wherein the granules of MU-IBDU have a crush strength of at least 1.5 kilogram (kg) per granule as tested with a Chatillon 225 series digital force tester.

4. The continuous method according to any of claims 1 to 3, wherein the fluid bed granulator comprises at least 2 chambers comprising a first chamber and a second chamber, and wherein the air is injected into the first chamber at a first temperature of from about 70 °C to 170 °C and the air is injected into the second chamber at a second temperature of from about 35 °C to 90 °C, wherein the second temperature is lower than the first temperature.

5. The continuous method according to any of claims 1 to 4, wherein the MU-IBDU slurry is injected into the fluid bed granulator at a temperature of from about 30 °C to about 110 °C.

6. The continuous method according to any of claims 1 to 5, further comprising mixing the MU-IBDU slurry with atomizing air prior to or at the time of injecting the MU-IBDU slurry into the fluid bed granulator.

7. The continuous method according to claim 6, wherein the atomizing air has a temperature of from about 70 °C to 140 °C.

8. The continuous method according to any of claims 1 to 7, wherein the granules of MU-IBDU have a ratio of MU to IBDU of from about 90:10 to about 25:75.

9. The continuous method according to any of claims 1 to 8, wherein the seed particles comprise MU-IBDU.

10. The continuous method according to any of claims 1 to 9, wherein the step of removing the granules of MU-IBDU from the fluid bed granulator comprises at least partially discharging the granules of MU-IBDU from the fluid bed granulator and passing the partially discharged granules of MU-IBDU through a screen to collect particles in a desired particle size range.

11. The continuous method according to claim 10, further comprising returning undersized granules of MU-IBDU to the fluid bed granulator as MU-IBDU seed particles.

12. The continuous method according to claim 10, further comprising crushing oversized granules of MU-IBDU to form smaller sized granules and returning the smaller sized granules to the fluid bed granulator as MU-IBDU seed particles.

13. A fertilizer composition comprising the granules of MU-IBDU formed according to the method of any of claims 1 to 12.

14. The fertilizer composition according to claim 13, further comprising an additional fertilizer, a micronutrient, a secondary nutrient, an organic additive, or a combination thereof.

15. The fertilizer composition according to claim 13 or 14, wherein the granules of MU-IBDU have a particle size of from about 1.5 mm to about 5 mm.
